(19) Europäisches Patentamt

European Patent Office

Office européen des brevets

(11) **EP 1 159 970 A2**

(12) **EUROPEAN PATENT APPLICATION**

(43) Date of publication:
**05.12.2001 Bulletin 2001/49**

(51) Int Cl.⁷: **A61K 45/06**, A61K 31/435,
A61P 3/04
// (A61K31/435, 31:365),
(A61K31/435, 31:135)

(21) Application number: **01304806.1**

(22) Date of filing: **31.05.2001**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **02.06.2000 US 208856 P**

(71) Applicant: **Pfizer Products Inc.**
**Groton, Connecticut 06340 (US)**

(72) Inventors:
• **Coe, Jotham W., Pfizer Global Res. & Devel.
Groton, Connecticut 06340 (US)**

• **O'Neill, Brian T., Pfizer Global Res. & Devel.
Groton, Connecticut 06340 (US)**
• **Sands, Steven B., Pfizer Global Res. & Devel.
Groton, Connecticut 06340 (US)**
• **Dow, Robert L.B., Pfizer Global Res. & Devel.
Groton, Connecticut 06340 (US)**
• **Harrigan, Edmund P., Pfizer Global Res. & Devel.
Groton, Connecticut 06340 (US)**
• **Watsky, Eric J., Pfizer Global Res. & Devel.
Groton, Connecticut 06340 (US)**

(74) Representative: **Eddowes, Simon et al
Urquhart-Dykes & Lord,
30 Welbeck Street
London W1G 8ER (GB)**

(54) **A pharmaceutical composition for the treatment of obesity or to facilitate or promote weight loss, comprising a nicotine receptor partial agonist and an anti-obesity agent**

(57) Pharmaceutical compositions are disclosed for the treatment of obesity, an overweight condition and compulsive overeating. The pharmaceutical compositions are comprised of a therapeutically effective combination of a nicotine receptor partial agonist and an anti-obesity agent or weight loss facilitator or promoter and a pharmaceutically acceptable carrier. The method of using these compounds is also disclosed.

EP 1 159 970 A2

**Description**

Background of the Invention

The present invention relates to pharmaceutical compositions for the treatment of obesity, compulsive overeating or to facilitate or promote weight loss in a mammal (e.g. human) comprising a nicotine receptor partial agonist (NRPA) and an anti-obesity or weight loss promoting agent. The term NRPA refers to all chemical compounds which bind at neuronal nicotinic acetylcholine specific receptor sites in mammalian tissue and elicit a partial agonist response. A partial agonist response is defined here to mean a partial, or incomplete functional effect in a given functional assay. Additionally, a partial agonist will also exhibit some degree of antagonist activity by its ability to block the action of a full agonist (Feldman, R.S., Meyer, J.S. & Quenzer, L.F. Principles of Neuropsychopharmacology, 1997; Sinauer Assoc. Inc.). The present invention may be used to treat mammals (e.g. humans) for obesity, an overweight condition or compulsive overeating with a decrease in the severity of unwanted side effects such as causing nausea and/or stomach upset.

Obesity is a major health risk that leads to increased mortality and incidence of Type 2 diabetes mellitus, hypertension and dyslipidemia. It is the second leading cause of preventable death in the United States, and contributes to >300,000 deaths per year. The estimated direct annual health cost associated with obesity is $70 billion, while the total overall cost to the U.S. economy has been estimated to be over $140 billion. In the U.S., more than 50% of the adult population is overweight, and almost ¼ of the population is considered to be obese (BMI greater than or equal to 30). Furthermore, the prevalence of obesity in the United States has increased by about 50% in the past 10 years. While the vast majority of obesity occurs in the industrialized world, particularly in US and Europe, the prevalence of obesity is also increasing in Japan. The prevalence of obesity in adults is 10%-25% in most countries of Western Europe. The rise in the incidence of obesity has promoted the WHO to recognize obesity as a significant disease. What is needed are orally active agents that induce sustained weight loss of 10-15% of initial body weight, due to selective loss of body fat in moderately obese patients. These orally active agents should increase energy expenditure, decrease food intake and partition energy away from adipose tissue. This degree of sustained weight loss would then improve comorbidities including hyperglycemia, hypertension and hyperlipidemia, all of which are exacerbated by obesity.

However, even though weight loss agents have therapeutic utility in the treatment of obesity, there are significant liabilities to the use of weight loss compounds. Specifically, many of these compounds that have been tested in humans can cause potentially serious side effects such as gastrointestinal complications including nausea, emesis, ulcers, constipation, flatulence, diarrhea, hypertension, respiratory depression, and psychological and physical dependence.

Summary of Invention

The present invention relates to a pharmaceutical composition for the treatment of obesity, compulsive overeating and/or to promote or facilitate weight loss comprising

> (a) a nicotine receptor partial agonist or a pharmaceutically acceptable salt thereof;
> (b) an anti-obesity agent or weight loss promoter or facilitator, or a pharmaceutically acceptable salt thereof; and
> (c) a pharmaceutically acceptable carrier;

wherein the active agents "a" and "b" above are present in amounts that render the composition effective in treating obesity, compulsive overeating and/or facilitating or promoting weight loss.

In a more specific embodiment of the invention the anti-obesity agent or weight loss promoter or facilitator is selected from Xenical™ (orlistat) or Meridia™ (sibutramine) and their pharmaceutically acceptable salts and optical isomers.

In another more specific embodiment of this invention, the nicotine receptor partial agonist is selected from:

> 9-bromo-1,2,3,4,5,6-hexahydro-1,5-methano-pyrido[1,2-a][1,5]diazocin-8-one;
> 9-chloro-1,2,3,4,5,6-hexahydro-1,5-methano-pyrido[1,2-a][1,5]diazocin-8-one;
> 9-fluoro-1,2,3,4,5,6-hexahydro-1,5-methano-pyrido[1,2-a][1,5]diazocin-8-one;
> 9-ethyl-1,2,3,4,5,6-hexahydro-1,5-methano-pyrido[1,2-a][1,5]diazocin-8-one;
> 9-methyl-1,2,3,4,5,6-hexahydro-1,5-methano-pyrido[1,2-a][1,5]diazocin-8-one;
> 9-phenyl-1,2,3,4,5,6-hexahydro-1,5-methano-pyrido[1,2-a][1,5]diazocin-8-one;
> 9-vinyl-1,2,3,4,5,6-hexahydro-1,5-methano-pyrido[1,2-a][1,5]diazocin-8-one;
> 9-bromo-3-methyl-1,2,3,4,5,6-hexahydro-1,5-methano-pyrido[1,2-a][1,5]diazocin-8-one;
> 3-benzyl-9-bromo-1,2,3,4,5,6-hexahydro-1,5-methano-pyrido[1,2-a][1,5]diazocin-8-one;
> 3-benzyl-9-chloro-1,2,3,4,5,6-hexahydro-1,5-methano-pyrido[1,2-a][1,5]diazocin-8-one;
> 9-acetyl-1,2,3,4,5,6-hexahydro-1,5-methano-pyrido[1,2a][1,5]diazocin-8-one;
> 9-iodo-1,2,3,4,5,6-hexahydro-1,5-methano-pyrido[1,2a][1,5]diazocin-8-one;
> 9-cyano-1,2,3,4,5,6-hexahydro-1,5-methano-pyrido[1,2a][1,5]diazocin-8-one;
> 9-ethynyl-1,2,3,4,5,6-hexahydro-1,5-methano-pyrido[1,2a][1,5]diazocin-8-one;

9-(2-propenyl)-1,2,3,4,5,6-hexahydro-1,5-methano-pyrido[1,2a][1,5]diazocin-8-one;

9-(2-propyl)- 1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

9-(4-fluorophenyl)-1,2,3,4,5,6-hexahydro-1,5-methano-pyrido[1,2a][1,5]diazocin-8-one;

9-(3-fluorophenyl)-1,2,3,4,5,6-hexahydro-1,5-methano-pyrido[1,2a][1,5]diazocin-8-one;

9-(35-difluorophenyl)-1,2,3,4,5,6-hexahydro-1,5-methano-pyrido[1,2a][1,5]diazocin-8-one;

9-(24-difluorophenyl)-1,2,3,4,5,6-hexahydro-1,5-methano-pyrido[1,2a][1,5]diazocin-8-one;

9-(2,5-difluorophenyl)-1,2,3,4,5,6-hexahydro-1,5-methano-pyrido[1,2a][1,5]diazocin-8-one;

6-methyl-5-oxo-6,13-diazatetracyclo$[9.3.1.0^{2,10}.0^{4,8}]$pentadeca-2(10),3,8-triene;

5-oxo-6,13-diazatetracyclo$[9.3.1.0^{2,10}.0^{4,8}]$pentadeca-2(10),3,8-triene;

6-oxo-5,7,13-triazatetracyclo$[9.3.1.0^{2,10}.0^{4,8}]$pentadeca-2(10),3,8-triene;

4,5-difluoro-10-aza-tricyclo$[6.3.1.0^{2,7}]$dodeca-2(7),3,5-triene;

5-fluoro-10-aza-tricyclo$[6.3.1.0^{2,7}]$dodeca-2(7),3,5-triene-4-carbonitrile;

4-ethynyl-5-fluoro-10-aza-tricyclo$[6.3.1.0^{2,7}]$dodeca-2(7),35-triene;

5-ethynyl-10-aza-tricyclo$[6.3.1.0^{2,7}]$dodeca-2(7),3,5-triene-4-carbonitrile;

6-methyl-5-thia-5-dioxa-6,13-diazatetracyclo$[9.3.1.0^{2,10}.0^{4,8}]$pentadeca-2(10),3,8-triene;

10-aza-tricyclo$[6.3.1.0^{2,7}]$dodeca-2(7),3,5-triene;

4-fluoro-10-aza-tricyclo$[6.3.1.0^{2,7}]$dodeca-2(7),3,5-triene;

4-methyl-10-aza-tricyclo$[6.3.1.0^{2,7}]$dodeca-2(7),3,5-triene;

4-trifluoromethyl-10-aza-tricyclo$[6.3.1.0^{2,7}]$dodeca-2(7),3,5-triene;

4-nitro-10-azatricyclo$[6.3.1.0^{2,7}]$dodeca-2(7),3,5-triene;

7-methyl-5,7,13-triazatetracyclo$[9.3.1.0^{2,10}.0^{4,8}]$pentadeca-2(10),3,5,8-tetraene;

6-methyl-5,7,13-triazatetracyclo$[9.3.1.0^{2,10}.0^{4,8}]$pentadeca-2(10),3,5,8-tetraene;

6,7-dimethyl-5,7,13-triazatetracyclo$[9.3.1.0^{2,10}.0^{4,8}]$pentadeca-2(10),3,5,8-tetraene;

6-methyl-7-phenyl-5,7,13-triazatetracyclo$[9.3.1.0^{2,10}.0^{4,8}]$pentadeca-2(10),3,5,8-tetraene;

6,7-dimethyl-5,8,14-triazatetracyclo$[10.3.1.0^{2,11}.0^{4,9}]$hexadeca-2(11),3,5,7,9-pentaene;

5,8,14-triazatetracyclo$[10.3.1.0^{2,11}.0^{4,9}]$hexadeca-2(11),3,5,7,9-pentaene;

14-methyl-5,8,14-triazatetracyclo$[10.3.1.0^{2,11}.0^{4,9}]$hexadeca-2(11),3,5,7,9-pentaene;

5-oxa-7,13-diazatetracyclo$[9.3.1.0^{2,10}.0^{4,8}]$pentadeca-2(10),3,6,8-tetraene;

6-methyl-5-oxa-7,13-diazatetracyclo$[3.3.1.0^{2,10}.0^{4,8}]$pentadeca-2(10),3,6,8-tetraene;

4-chloro-10-azatricyclo$[6.3.1.0^{2,7}]$dodeca-2(7),3,5-triene;

10-azatricyclo$[6.3.1.0^{2,7}]$dodeca-2(7),3,5-trien-4-yl cyanide;

1-(10-azatricyclo$[6.3.1.0^{2,7}]$dodeca-2(7),3,5-trien-4-yl)-1-ethanone;

10-azatricyclo$[6.3.1.0^{2,7}]$dodeca-2(7),3,5-trien-4-ol;

7-methyl-5-oxa-6,13-diazatetracyclo$[9.3.1.0^{2,10}.0^{4,8}]$pentadeca-2,4(8),6,9-tetraene;

4,5-dichloro-10-azatricyclo$[6.3.1.0^{2,7}]$dodeca-2(7),3,5-triene;

11-azatricyclo$[7.3.1.0^{2,7}]$trideca-2(7),3,5-triene-5-carbonitrile;

1-[11-azatricyclo$[7.3.1.0^{2,7}]$trideca-2(7),3,5-trien-5-yl]-1-ethanone;

1-[11-azatricyclo$[7.3.1.0^{2,7}]$trideca-2(7),3,5-trien-5-yl]-1-propanone;

4-fluoro-11-azatricyclo$[7.3.1.0^{2,7}]$trideca-2(7),3,5-triene-5-carbonitrile;

5-fluoro-11-azatricyclo$[7.3.1.0^{2,7}]$trideca-2(7)3,5-triene-4-carbonitrile;

6-methyl-7-thia-5,14-diazatetracyclo$[10.3.1.0^{2,10}.0^{4,8}]$hexadeca-2(10),3,5,8-tetraene;

6-methyl-5,7,14-triazatetracyclo$[10.3.1.0^{2,10}.0^{4,8}]$hexadeca-2(10),3,5,8-tetraene;

6,7-dimethyl-5,7,14-triazatetracyclo$[10.3.1.0^{2,10}.0^{4,8}]$hexadeca-2(10),3,5,8-tetraene;

5,7,14-triazatetracyclo$[10.3.1.0^{2,10}.0^{4,8}]$hexadeca-2(10),3,5,8-tetraene;

5,6-dimethyl-5,7,14-triazatetracyclo$[10.3.1.0^{2,10}.0^{4,8}]$hexadeca-2(10),3,6,8-tetraene;

5-methyl-5,7,14-triazatetracyclo$[10.3.1.0^{2,10}.0^{4,8}]$hexadeca-2(10),3,6,8-tetraene;

6-(trifluoromethyl)-7-thia-5,14-diazatetracyclo$[10.3.1.0^{2,10}.0^{4,8}]$hexadeca-2(10),3,5,8-tetraene;

5,8,15-triazatetracyclo$[11.3.1.0^{2,11}.0^{4,9}]$heptadeca-2(11),3,5,7,9-pentaene;

7-methyl-5,8,15-triazatetracyclo$[11.3.1.0^{2,11}.0^{4,9}]$heptadeca-2(11),3,5,7,9-pentaene;

6-methyl-5,8,15-triazatetracyclo$[11.3.1.0^{2,11}.0^{4,9}]$heptadeca-2(11),3,5,7,9-pentaene;

6,7-dimethyl-5,8,15-triazatetracyclo$[11.3.1.0^{2,11}.0^{4,9}]$heptadeca-2(11),3,5,7,9-pentaene;

7-oxa-5,14-diazatetracyclo$[10.3.1.0^{2,10}.0^{4,8}]$hexadeca-2(10),3,5,8-tetraene;

6-methyl-7-oxa-5,14-diazatetracyclo$[10.3.1.0^{2,10}.0^{4,8}]$hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene; and

6-hydroxy-5-methoxy-11-aza-tricyclo[7.3.1.0$^{2,7}$]trideca-2(7),3,5-triene and

their pharmaceutically acceptable salts and their optical isomers.

Preferably, the nicotine receptor partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(26-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo[9.3.1.0$^{2,10}$.0$^{4,8}$]pentadeca-2(10),3,8-triene;

4-fluoro-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

6-methyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

6-fluoro-11-aza-tricyclo[7.3.1.0$^{2,7}$]trideca-2(7),3,5-triene; and

11-aza-tricyclo[7.3.1.0$^{2,7}$]trideca-2(7),3,5-trien-5-ol and

their pharmaceutically acceptable salts and their optical isomers.

The present invention also relates to a method of treating obesity overeating, and/or facilitating or promoting weight loss in a mammal comprising administering to said mammal respectively an anti-obesity attenuating effective amount of a pharmaceutical composition comprising

(a) a nicotine receptor partial agonist or a pharmaceutically acceptable salt thereof; and
(b) an anti-obesity agent or a weight loss promoter or facilitator or a pharmaceutically acceptable salt thereof;

wherein the active ingredients (a) and (b) are present in amounts that render the composition effective in the treatment of obesity, compulsive overeating or an overweight condition.

In another more specific embodiment of this invention the nicotine receptor partial agonist is selected from:

9-bromo-1,2,3,4,5,6-hexahydro-1,5-methano-pyrido[1,2-a][1,5]diazocin-8-one;
9-chloro-1,2,3,4,5,6-hexahydro-1,5-methano-pyrido[1,2-a][1,5]diazocin-8-one;
9-fluoro-1,2,3,4,5,6-hexahydro-1,5-methano-pyrido[1,2-a][1,5]diazocin-8-one;
9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)- 1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(3,5-difluorophenyl)-1,2,3,4,5,6-hexahydro-1,5-methano-pyrido[1,2a][1,5]diazocin-8-one;
9-(24-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]dodeca-2(7),3,5-triene;
5-ethynyl-10-aza-tricyclo[6.3.1.0$^{2,7}$]dodeca-2(7),3,5-triene-4-carbonitrile;
6-methyl-5-thia-5-dioxa-6,13-diazatetracyclo[9.3.1.0$^{2,10}$.0$^{4,8}$]pentadeca-2(10),3,8-triene;
10-aza-tricyclo[6.3.1.0$^{2,7}$]dodeca-2(7),3,5-triene;
4-fluoro-10-aza-tricyclo[6.3.1.0$^{2,7}$]dodeca-2(7),3,5-triene;
4-methyl-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7),3,5-triene;
7-methyl-5,7,13-triazatetracyclo[9.3.1.0$^{2,10}$.0$^{4,8}$]pentadeca-2(10),3,5,8-tetraene;
6-methyl-5,7,13-triazatetracyclo[9.3.1.0$^{2,10}$.0$^{4,8}$]pentadeca-2(10),3,5,8-tetraene;
6,7-dimethyl-5,7,13-triazatetracyclo[9.3.1.0$^{2,10}$.0$^{4,8}$]pentadeca-2(10),3,5,8-tetraene;
6-methyl-7-phenyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
14-methyl-5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10)3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene; and

6-hydroxy-5-methoxy-11-aza-tricyclo[7.3.1.0$^{2,7}$]trideca-2(7),3,5-triene and

their pharmaceutically acceptable salts and their optical isomers.

Preferably, the nicotine receptor partial agonist is selected from:

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1 ,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo[9.3.1.0$^{2,10}$.0$^{4,8}$]pentadeca-2(10),3,8-triene;
4-fluoro-10-aza-tricyclo[6.3.1.0$^{2,7}$]dodeca-2(7),3,5-triene;
4-trifluoromethyl-10-aza-tricyclo[6.3.1.0$^{2,7}$]dodeca-2(7),3,5-triene;
4-nitro-10-azatricyclo[6.3.1.0$^{2,7}$]dodeca-2(7)3,5-triene;
6-methyl-5,7,13-triazatetracyclo[9.3.1.0$^{2,10}$.0$^{4,8}$]pentadeca-2(10),3,5,8-tetraene;
6,7-dimethyl-5,8,14-triazatetracyclo[10.3.1.0$^{2,11}$.0$^{4,9}$]hexadeca-2(11),3,5,7,9-pentaene;
5,8,14-triazatetracyclo[10.3.1.0$^{2,11}$.0$^{4,9}$]hexadeca-2(11),3,5,7,9-pentaene;
5-oxa-7,13-diazatetracyclo[9.3.1.0$^{2,10}$.0$^{4,8}$]pentadeca-2(10),3,6,8-tetraene;
6-methyl-5-oxa-7,13-diazatetracyclo[9.3.1.0$^{2,10}$.0$^{4,8}$]pentadeca-2(10),3,6,8-tetraene;
10-azatricyclo[6.3.1.0$^{2,7}$]dodeca-2(7),3,5-trien-4-yl cyanide;
1-(10-azatricyclo[6.3.1.0$^{2,7}$]dodeca-2(7),3,5-trien-4-yl)-1-ethanone;
11-azatricyclo[7.3.1.0$^{2,7}$]trideca-2(7),3,5-triene-5-carbonitrile;
1-[11-azatricyclo[7.3.1.0$^{2,7}$]trideca-2(7),3,5-trien-5-yl]-1-ethanone;
1-[11-azatricyclo[7.3.1.0$^{2,7}$]trideca-2(7),3,5-trien-5-yl]-1-propanone;
4-fluoro-11-azatricyclo[7.3.1.0$^{2,7}$]trideca-2(7),3,5-triene-5-carbonitrile;
5-fluoro-11-azatricyclo[7.3.1.0$^{2,7}$]trideca-2(7),3,5-triene-4-carbonitrile;
6-methyl-7-thia-5,14-diazatetracyclo[10.3.1.0$^{2,10}$.0$^{4,8}$]hexadeca-2(10),3,5,8-tetraene;
6-methyl-5,7,14-triazatetracyclo[10.3.1.0$^{2,10}$.0$^{4,8}$]hexadeca-2(10),3,5,8-tetraene;
6,7-dimethyl-5,7,14-triazatetracyclo[10.3.1.0$^{2,10}$.0$^{4,8}$]hexadeca-2(10),3,5,8-tetraene;
6-methyl-7-oxa-5,14-diazatetracyclo[10.3.1.0$^{2,10}$.0$^{4,8}$]hexadeca-2(10),3,5,8-tetraene;
6-methyl-5-oxa-7,14-diazatetracyclo[10.3.1.0$^{2,10}$.0$^{4,8}$]hexadeca-2(10),3,6,8-tetraene;
5,6-difluoro-11-aza-tricyclo[7.3.1.0$^{2,7}$]trideca-2,4,6-triene;
6-trifluoromethyl-11-aza-tricyclo[7.3.1.0$^{2,7}$]trideca-2,4,6-triene;
6-methoxy-11-aza-tricyclo[7.3.1.0$^{2,7}$]trideca-2(7),3,5-triene;
6-fluoro-11-aza-tricyclo[7.3.1.0$^{2,7}$]trideca-2(7),3,5-triene; and
11-aza-tricyclo[7.3.1.0$^{2,7}$]trideca-2(7),3,5-trien-5-ol;

and the pharmaceutically acceptable salts stereoisomers (including optical isomers), solvates and hydrates of the foregoing compounds.

The above NRPA's and others are referred to, along with methods for their synthesis in World Patent Applications WO 98/18798, WO 99/35131 and WO 99/55680, which were published, respectively on May 7, 1998, July 15, 1999 and November 4, 1999. The foregoing applications are owned in common with the present application and are incorporated herein by reference in their entireties. These compounds can be used in combination with an anti-obesity agent or a weight loss promoter in order to treat obesity or facilitate or promote weight loss.

In another more specific embodiment, the anti-obesity agent and/or weight loss promoter or facilitator is selected from Xenical™ (orlistat) or Meridia™ (sibutramine) and their pharmaceutically acceptable salts, stereo isomers (including optical isomers), hydrates and solvates.

The invention also relates to pharmaceutical composition for treating a disorder or condition selected from the group consisting of disorders and conditions in which obesity or an overweight condition predominates, including Type 2 diabetes mellitus, hypertension, dyslipidemia and increased mortality in a mammal, including a human, comprising;

(a) a nicotine receptor partial agonist or a pharmaceutically acceptable salt thereof,
(b) an anti-obesity agent or weight-loss promoter or facilitator or a pharmaceutically acceptable salt thereof;
(c) a pharmaceutically acceptable carrier;

wherein the active ingredients (a) and (b) above are present in amounts that render the composition effective in treating obesity, compulsive over-eating or an overweight condition.

The invention also relates to a method of treating a disorder or condition selected from the group of disorders and condition in which obesity or an overweight condition predominates, including Type 2 diabetes mellitus, hypertension, dyslipidemia, and increased mortality in a mammal, including a human, comprising administering to said mammal;

(a) a nicotine receptor partial agonist or a pharmaceutically acceptable salt thereof; and
(b) an anti-obesity agent or weight loss promoter or facilitator or a pharmaceutically acceptable salt thereof;

wherein the active ingredients (a) and (b) above are present in amounts that render the combination of the two active agents effective in treating such disorder or condition.

The nicotine receptor partial agonist and the anti obesity agent or weight loss promoter or facilitator can be administered substantially simultaneously.

The term "treating" as used herein, refers to reversing, alleviating, inhibiting or slowing the progress of, or preventing the disorder or condition to which such term applies, or one or more symptoms of such disorder or condition. The term "treatment", as used herein, refers to the act of treating, as "treating" is defined immediately above.

Detailed Description of the Invention

In combination with the NRPA, the invention includes an anti-obesity agent or a weight loss facilitator, or a pharmaceutically acceptable salt of compounds such as Xenical™ (orlistat) or Meridia™ (sibutramine).

A nicotine partial agonist combined with an anti-obesity agent may facilitate weight loss while reducing the incidence of undesirable side effects. Nicotine has long been appreciated to have anorectic properties, but its use has been limited by a poor spectrum of activity, side effects, and less efficacy than anti-obesity agents. This may be due to lack of specificity of nicotine for neuromuscular, ganglionic, and central nervous system receptors. The development of nicotine partial agonists with specific receptor subtype affinities is an approach to potentially reduce side effects and enhance efficacy.

Over the past several years it has become clear that obesity has an important genetic component. Scientific investigation of monogenic rodent models of obesity has revealed novel mechanisms important in the regulation of body weight homeostasis including leptin or a leptin receptor. Several of these genes are now the targets of drug discovery efforts. Human obesity, however, is rarely due to monogenic causes but rather is a result of complex multigenic and environmental interactions. Despite the important role of genetics in the predisposition to obesity in humans, the obese phenotype results only after prolonged positive energy balance due to excess energy consumption or insufficient energy expenditure. Conversely, weight loss can only take place when energy expenditure exceeds energy intake over an extended interval. Weight loss can be achieved by stimulating energy expenditure, decreasing caloric intake, decreasing energy absorption and/or favorable partitioning of energy to skeletal muscle where it is converted to muscle mass as opposed to adipose tissue where it is stored. The goal is to achieve sustained weight loss of 5-15% or greater leading to an improvement of glycemic control up to a 2% decrease in HbA1c in diabetics, reductions in diastolic blood pressure to 90 mm Hg in hypertensives, and/or decreases in LDL cholesterol by $\geq$ 15% in hyperlipidemic patients.

The particular NRPA compounds listed above, which can be employed in the methods and pharmaceutical compositions of this invention, can be made by processes known in the chemical arts, for example by the methods described in WO 9818798 A1, WO 9935131-A1 and WO9955680-A1. Some of the preparation methods useful for making the compounds of this invention may require protection of remote functionality (i.e., primary amine, secondary amine, carboxyl). The need for such protection will vary depending on the nature of the remote functionality and the conditions of the preparation methods. The need for such protection is readily determined by one skilled in the art, and is described in examples carefully described in the above cited applications. The starting materials and reagents for the NRPA compounds employed in this invention are also readily available or can be easily synthesized by those skilled in the art using conventional methods of organic synthesis. Some of the compounds used herein are related to, or are derived from compounds found in nature and accordingly many such compounds are commercially available or are reported in the literature or are easily prepared from other commonly available substances by methods which are reported in the literature.

Some of the NRPA compounds employed in this invention are ionizable at physiological conditions. Thus, for example some of the compounds of this invention are acidic and they form a salt with a pharmaceutically acceptable cation. The use of all such salts are within the scope of the pharmaceutical compositions and methods this invention and they can be prepared by conventional methods. For example, they can be prepared simply by contacting the acidic and basic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

In addition, some of the NRPA compounds employed in this invention are basic, and they form a salt with a pharmaceutically acceptable acid. All such salts are within the scope of this invention and they can be prepared by conventional methods. For example, they can be prepared simply by contacting the basic and acidic entities, usually in a stoichiometric ratio, in either an aqueous, non-aqueous or partially aqueous medium, as appropriate. The salts are recovered either by filtration, by precipitation with a non-solvent followed by filtration, by evaporation of the solvent, or, in the case of aqueous solutions, by lyophilization, as appropriate.

The utility of the NRPA compounds employed in the present invention as medicinal agents in the treatment of obesity, compulsive overeating, and an overweight condition in mammals (e.g. humans) is demonstrated by the activity of the compounds of this invention in conventional assays and, in particular the assays described below. Such assays also provide a means whereby the activities of the compounds of this invention can be compared between themselves and with the activities of other known compounds. The results of these comparisons are useful for determining dosage levels in mammals, including humans, for the treatment of such diseases.

Administration of the compositions of this invention can be via any method which delivers a compound of this invention systemically and/or locally. These methods which include oral routes and transdermal routes, etc. Generally, the compounds of this invention are administered orally, but parenteral administration may be utilized (e.g., intravenous, intramuscular, subcutaneous or intramedullary). The two different compounds of this invention can be co-administered simultaneously or sequentially in any order, or single pharmaceutical composition comprising a NRPA as described above and an analgesic agent as described above in a pharmaceutically acceptable carrier can be administered.

Procedures

Receptor binding assay: The effectiveness of the active compounds in suppressing nicotine binding to specific receptor sites is determined by the following procedure which is a modification of the methods of Lippiello, P. M. and Fernandes, K. G. (in The Binding of L-[³H]Nicotine To A Single Class of High-Affinity Sites in Rat Brain Membranes, Molecular Pharm., 29, 448-54, (1986)) and Anderson, D. J. and Arneric, S. P. (in Nicotinic Receptor Binding of ³H-Cystisine, ³H-Nicotine and ³H-Methylcarmbamylcholine In Rat Brain, European J. Pharm., 253, 261-67 (1994)). Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.* The rats were killed by decapitation. Brains were removed immediately following decapitation. Membranes were prepared from brain tissue according to the methods of Lippiello and Fernandez (Molec Pharmacol, 29, 448-454, (1986) with some modifications. Whole brains were removed, rinsed with ice-cold buffer, and homogenized at 0° in 10 volumes of buffer (w/v) using a Brinkmann Polytron™, setting 6, for 30 seconds. The buffer consisted of 50 mM Tris HCl at a pH of 7.5 at room temperature. The homogenate was sedimented by centrifugation (10 minutes; 50,000 x g; 0° to 4°C). The supernatant was poured off and the membranes were gently resuspended with the Polytron and centrifuged again (10 minutes; 50,000 x g; 0 to 4°C. After the second centrifugation, the membranes were resuspended in assay buffer at a concentration of 1.0g/100mL. The composition of the standard assay buffer was 50 mM Tris HCl, 120 mM NaCl, 5 mM KCl, 2 mM MgCl$_2$, 2 mM CaCl$_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50µL of vehicle, blank, or test compound solution, respectively. To each tube was added 200µL of [³H]-nicotine in assay buffer followed by 750µL of the membrane suspension. The final concentration of nicotine in each tube was 0.9 nM. The final concentration of cytisine in the blank was 1µM. The vehicle consisted of deionized water containing 30µL of 1 N acetic acid per 50 mL of water. The test compounds and cytisine were dissolved in vehicle. Assays were initiated by vortexing after addition of the membrane suspension to the tube. The samples were incubated at 0° to 4° C in an iced shaking water bath. Incubations were terminated by rapid filtration under vacuum through Whatman GF/B™ glass fiber filters using a Brandel™ multi-manifold tissue harvester. Following the initial filtration of the assay mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations: Specific binding (C) to the membrane is the difference between total binding in the samples containing vehicle only and membrane (A) and non-specific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) - (B).

$$\% \text{ Inhibition} = (1-((E)/(C))) \text{ times } 100.$$

The compounds of the invention that were tested in the above assay exhibited IC$_{50}$ values of less than 10µM.

The amount and timing of compounds administered will, of course, be based on the judgement of the prescribing physician. Thus, because of patient to patient variability, the dosages given below are a guideline and the physician may titrate doses of the agent to achieve the activity that the physician considers appropriate for the individual patient. In considering the degree of activity desired, the physician must balance a variety of factors such as cognitive function, age of the patient, presence of preexisting disease, as well as presence of other diseases (e.g., cardiovascular). The following paragraphs provide preferred dosage ranges for the various components of this invention (based on average human weight of 70 kg).

In general, an effective dosage for the NRPA in the range of 0.01 to 200 mg/kg/day, preferably 0.05 to 10.0 mg/kg/day.

In particular, an effective dosage for Xenical™ (orlistat) when used in the combination compositions and methods of this invention, is in the range of 1.0 - 5.0 mg/kg/day.

In particular, an effective dosage for Meridia™ (sibutramine) when used in the combination compositions and methods of this invention, is in the range of 0.01 - 0.2 mg/kg/day.

The compositions of the present invention are generally administered in the form of a pharmaceutical composition comprising at least one of the compounds of this invention together with a pharmaceutically acceptable vehicle or diluent. Thus, the compounds of this invention can be administered individually or together in any conventional oral, parenteral or transdermal dosage form.

For oral administration a pharmaceutical composition can take the form of solutions, suspensions, tablets, pills, capsules, powders, and the like. Tablets containing various excipient such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples, see Remington's Pharmaceutical Sciences, Mack Publishing Company, Easter, Pa., 15th Edition (1975).

Pharmaceutical compositions according to the invention may contain 0.1%-95% of the compound(s) of this invention, preferably 1%-70%. In any event, the composition or formulation to be administered will contain a quantity of a compound(s) according to the invention in an amount effective to treat the obesity or compulsive overeating of the subject being treated.

## Claims

1. A pharmaceutical composition for the treatment of obesity, compulsive overeating, or to promote or facilitate weight loss comprising:

   (a) a nicotine receptor partial agonist or a pharmaceutically acceptable salt thereof;
   (b) an anti-obesity agent or a weight loss promoter or facilitator or pharmaceutically acceptable salt thereof; and
   (c) a pharmaceutically acceptable carrier;

   wherein the active agents "a" and "b" above are present in amounts that render the composition effective in treating obesity, compulsive overeating or promoting or facilitating weight loss.

2. The pharmaceutical composition according to Claim 1, wherein said anti-obesity agent or weight loss promoter or facilitator is selected from Xenical™ (orlistat) and Meridia™ (sibutramine) and their pharmaceutically active salts.

3. The pharmaceutically composition according to Claim 1, wherein said nicotine receptor partial agonist is selected from:

   9-bromo-1,2,3,4,5,6-hexahydro-1,5-methano-pyrido[1,2-a][1,5]diazocin-8-one;
   9-chloro-1,2,3,4,5,6-hexahydro-1,5-methano-pyrido[1,2-a][1,5]diazocin-8-one;
   9-fluoro-1,2,3,4,5,6-hexahydro-1,5-methano-pyrido[1,2-a][1,5]diazocin-8-one;
   9-ethyl-1,2,3,4,5,6-hexahydro-1,5-methano-pyrido[1,2-a][1,5]diazocin-8-one;
   9-methyl-1,2,3,4,5,6-hexahydro-1,5-methano-pyrido[1,2-a][1,5]diazocin-8-one;
   9-phenyl-1,2,3,4,5,6-hexahydro-1,5-methano-pyrido[1,2-a][1,5]diazocin-8-one;
   9-vinyl-1,2,3,4,5,6-hexahydro-1,5-methano-pyrido[1,2-a][1,5]diazocin-8-one;
   9-bromo-3-methyl-1,2,3,4,5,6-hexahydro-1,5-methano-pyrido[1,2-a][1,5]diazocin-8-one;
   3-benzyl-9-bromo-1,2,3,4,5,6-hexahydro-1,5-methano-pyrido[1,2-a][1,5]diazocin-8-one;
   3-benzyl-9-chloro-1,2,3,4,5,6-hexahydro-1,5-methano-pyrido[1,2-a][1,5]diazocin-8-one;
   9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-pyrido[1,2a][1,5]diazocin-8-one;

9-ethynyl-1,2,3,4,5,6-hexahydro-1,5-methano-pyrido[1,2a][1,5]diazocin-8-one;

9-(2-propenyl)-1,2,3,4,5,6-hexahydro-1,5-methano-pyrido[1,2a][1,5]diazocin-8-one;

9-(2-propyl)- 1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1 ,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

9-(4-fluorophenyl)-1,2,3,4,5,6-hexahydro-1,5-methano-pyrido[1,2a][1,5]diazocin-8-one;

9-(3-fluorophenyl)-1,2,3,4,5,6-hexahydro-1,5-methano-pyrido[1,2a][1,5]diazocin-8-one;

9-(3,5-difluorophenyl)-1,2,3,4,5,6-hexahydro-1,5-methano-pyrido[1,2a][1,5]diazocin-8-one;

9-(2,4-difluorophenyl)-1,2,3,4,5,6-hexahydro-1,5-methano-pyrido[1,2a][1,5]diazocin-8-one;

9-(2,5-difluorophenyl)-1,2,3,4,5,6-hexahydro-1,5-methano-pyrido[1,2a][1,5]diazocin-8-one;

6-methyl-5-oxo-6,13-diazatetracyclo [9.3.1.$0^{2,10}$.$0^{4,8}$]pentadeca-2(10),3,8-triene;

5-oxo-6,13-diazatetracyclo[9.3.1.$0^{2,10}$.$0^{4,8}$] pentadeca-2(10),3,8-triene;

6-oxo-5,7,13-triazatetracyclo[9.3.1.$0^{2,10}$.$0^{4,8}$] pentadeca-2(10),3,8-triene;

4,5-difluoro-10-aza-tricyclo[6.3.1.$0^{2,7}$]dodeca-2(7),3,5-triene;

5-fluoro-10-aza-tricyclo[6.3.1.$0^{2,7}$]dodeca-2 (7),3,5-triene-4-carbonitrile;

4-ethynyl-5-fluoro-10-aza-tricyclo[6.3.1.$0^{2,7}$] dodeca-2(7),3,5-triene;

5-ethynyl-10-aza-tricyclo[6.3.1.$0^{2,7}$]dodeca-2 (7),3,5-triene-4-carbonitrile;

6-methyl-5-thia-5-dioxa-6,13-diazatetracyclo [9.3.1.$0^{2,10}$.$0^{4,8}$]pentadeca-2(10),3,8-triene;

10-aza-tricyclo[6.3.1.$0^{2,7}$]dodeca-2(7), 3,5-triene;

4-fluoro-10-aza-tricyclo[6.3.1.$0^{2,7}$]dodeca-2 (7),35-triene;

4-methyl-10-aza-tricyclo[6.3.1.$0^{2,7}$]dodeca-2 (7),3,5-triene;

4-trifluoromethyl-10-aza-tricyclo[6.3.1.$0^{2,7}$]dodeca-2(7),3,5-triene;

4-nitro-10-azatricyclo[6.3.1.$0^{2,7}$]dodeca-2(7), 3,5-triene;

7-methyl-5,7,13-triazatetracyclo[9.3.1.$0^{2,10}$.$0^{4,8}$]pentadeca-2(10),3,5,8-tetraene;

6-methyl-5,7,13-triazatetracyclo[9.3.1.$0^{2,10}$.$0^{4,8}$]pentadeca-2(10),3,5,8-tetraene;

6,7-dimethyl-5,7,13-triazatetracyclo [9.3.1.$0^{2,10}$.$0^{4,8}$]pentadeca-2(10), 3,5,8-tetraene;

6-methyl-7-phenyl-5,7,13-triazatetracyclo [9.3.1.$0^{2,10}$.$0^{4,8}$]pentadeca-2(10), 3,5,8-tetraene;

6,7-dimethyl-5,8,14-triazatetracyclo [10.3.1.$0^{2,11}$.$0^{4,9}$]hexadeca-2(11),3,5,7,9-pentaene;

5,8,14-triazatetracyclo[10.3.1.$0^{2,11}$.$0^{4,9}$]hexadeca-2(11),3,5,7,9-pentaene;

14-methyl-5,8,14-triazatetracyclo[10.3.1.$0^{2,11}$.$0^{4,9}$]hexadeca-2(11),3,5,7,9-pentaene;

5-oxa-7,13-diazatetracyclo[9.3.1.$0^{2,10}$.$0^{4,8}$] pentadeca-2(10),3,6,8-tetraene;

6-methyl-5-oxa-7,13-diazatetracyclo [9.3.1.$0^{2,10}$.$0^{4,8}$]pentadeca-2(10), 3,6,8-tetraene;

4-chloro-10-azatricyclo[6.3.1.$0^{2,7}$]dodeca-2 (7),3,5-triene;

10-azatricyclo[6.3.1.$0^{2,7}$]dodeca-2(7),3,5-trien-4-yl cyanide;

1-(10-azatricyclo[6.3.1.$0^{2,7}$]dodeca-2(7), 3,5-trien-4-yl)-1-ethanone;

10-azatricyclo[6.3.1.$0^{2,7}$]dodeca-2(7),3,5-trien-4-ol;

7-methyl-5-oxa-6,13-diazatetracyclo [9.3.1.$0^{2,10}$.$0^{4,8}$]pentadeca-2,4(8), 6,9-tetraene;

4,5-dichloro-10-azatricyclo[6.3.1.$0^{2,7}$]dodeca-2(7),3,5-triene;

11-azatricyclo[7.3.1.$0^{2,7}$]trideca-2(7), 3,5-triene-5-carbonitrile;

1-[11-azatricyclo[7.3.1.$0^{2,7}$]trideca-2(7),3,5-trien-5-yl]-1-ethanone;

1-[11-azatricyclo[7.3.1.$0^{2,7}$]trideca-2(7),3,5-trien-5-yl]-1-propanone;

4-fluoro-11-azatricyclo[7.3.1.$0^{2,7}$]trideca-2(7), 3,5-triene-5-carbonitrile;

5-fluoro-11-azatricyclo[7.3.1.$0^{2,7}$]trideca-2(7), 3,5-triene-4-carbonitrile;

6-methyl-7-thia-5,14-diazatetracyclo [10.3.1.$0^{2,10}$.$0^{4,8}$]hexadeca-2(10), 3,5,8-tetraene;

6-methyl-5,7,14-triazatetracyclo[10.3.1.$0^{2,10}$.$0^{4,8}$]hexadeca-2(10),3,5,8-tetraene;

6,7-dimethyl-5,7,14-triazatetracyclo [10.3.1.$0^{2,10}$.$0^{4,8}$]hexadeca-2(1 0), 3,5,8-tetraene;

5,7,14-triazatetracyclo[10.3.1.$0^{2,10}$.$0^{4,8}$]hexadeca-2(10),3,5,8-tetraene;

5,6-dimethyl-5,7,14-triazatetracyclo [10.3.1.$0^{2,10}$.$0^{4,8}$]hexadeca-2(10), 3,6,8-tetraene;

5-methyl-5,7,14-triazatetracyclo[10.3.1.$0^{2,10}$.$0^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10),3,5,8-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene;

6-hydroxy-5-methoxy-11-aza-tricyclo[7.3.1.0$^{2,7}$]trideca-2(7),3,5-triene; and

their pharmaceutically acceptable salts and their optical isomers.

4. The pharmaceutical composition according to Claim 3 wherein said nicotine receptor partial agonist is selected from:

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[1,2a][1,5]diazocin-8-one;

6-methyl-5-thia-5-dioxa-6,13-diazatetracyclo[9.3.1.0$^{2,10}$.0$^{4,8}$]pentadeca-2(10),3,8-triene;

4-fluoro-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

6-methyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(1),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol, and their pharmaceutically acceptable salts and their optical isomers thereof.

5. A method of treating obesity, or promoting or facilitating weight loss in a mammal comprising administering to said mammal:

   a. a nicotine receptor partial agonist or a pharmaceutically acceptable salt thereof; and
   b. an anti-obesity agent or weight loss promoter or facilitator, or a pharmaceutically acceptable salt thereof,

   wherein the active ingredients (a) and (b) are adminstered in amounts that render the combination of the two active agent effective in the treatment of obesity, or in promoting or facilitating weight loss.

6. The method of claim 5, wherein the anti-obesity agent or weight loss facilitator is selected from, Xenical™ (orlistat) or Meridia™ (sibutramine) and their pharmaceutically active salts.

7. The method according to claim 5, wherein the nicotine partial agonist is selected from

   9-bromo-1,2,3,4,5,6-hexahydro-1,5-methano-pyrido[1,2-a][1,5]diazocin-8-one;
   9-chloro-1,2,3,4,5,6-hexahydro-1,5-methano-pyrido[1,2-a][1,5]diazocin-8-one;
   9-fluoro-1,2,3,4,5,6-hexahydro-1,5-methano-pyrido[1,2-a][1,5]diazocin-8-one;

9-ethyl-1,2,3,4,5,6-hexahydro-1,5-methano-pyrido[1,2-a][1,5]diazocin-8-one;
9-methyl-1,2,3,4,5,6-hexahydro-1,5-methano-pyrido[1,2-a][1,5]diazocin-8-one;
9-phenyl-1,2,3,4,5,6-hexahydro-1,5-methano-pyrido[1,2-a][1,5]diazocin-8-one;
9-vinyl-1,2,3,4,5,6-hexahydro-1,5-methano-pyrido[1,2-a][1,5]diazocin-8-one;
9-bromo-3-methyl-1,2,3,4,5,6-hexahydro-1,5-methano-pyrido[1,2-a][1,5]diazocin-8-one;
3-benzyl-9-bromo-1,2,3,4,5,6-hexahydro-1,5-methano-pyrido[1,2-a][1,5]diazocin-8-one;
3-benzyl-9-chloro-1,2,3,4,5,6-hexahydro-1,5-methano-pyrido[1,2-a][1,5]diazocin-8-one;
9-acetyl-1,2,3,4,5,6-hexahydro-1,5-methano-pyrido[1,2a][1,5]diazocin-8-one;
9-iodo-1,2,3,4,5,6-hexahydro-1,5-methano-pyrido[1,2a][1,5]diazocin-8-one;
9-cyano-1,2,3,4,5,6-hexahydro-1,5-methano-pyrido[1,2a][1,5]diazocin-8-one;
9-ethynyl-1,2,3,4,5,6-hexahydro-1,5-methano-pyrido[1,2a][1,5]diazocin-8-one;
9-(2-propenyl)-1,2,3,4,5,6-hexahydro-1,5-methano-pyrido[1,2a][1,5]diazocin-8-one;
9-(2-propyl)-1,2,3,4,5,6-hexahydro-1,5-methano-pyrido[1,2a][1,5]diazocin-8-one;
9-carbomethoxy-1,2,3,4,5,6-hexahydro-1,5-methano-pyrido[1,2a][1,5]diazocin-8-one;
9-carboxyaldehyde-1,2,3,4,5,6-hexahydro-1,5-methano-pyrido[1,2a][1,5]diazocin-8-one;
9-(2,6-difluorophenyl)-1,2,3,4,5,6-hexahydro-1,5-methano-pyrido[1,2a][1,5]diazocin-8-one;
9-phenyl-1,2,3,4,5,6-hexahydro-1,5-methano-pyrido[1,2a][1,5]diazocin-8-one;
9-(2-fluorophenyl)-1,2,3,4,5,6-hexahydro-1,5-methano-pyrido[1,2a][1,5]diazocin-8-one;
9-(4-fluorophenyl)-1,2,3,4,5,6-hexahydro-1,5-methano-pyrido[1,2a][1,5]diazocin-8-one;
9-(3-fluorophenyl)-1,2,3,4,5,6-hexahydro-1,5-methano-pyrido[1,2a][1,5]diazocin-8-one;
9-(35-difluorophenyl)-1,2,3,4,5,6-hexahydro-1,5-methano-pyrido[1,2a][1,5]diazocin-8-one;
9-(2,4-difluorophenyl)-1,2,3,4,5,6-hexahydro-1,5-methano-pyrido[1,2a][1,5]diazocin-8-one;
9-(2,5-difluorophenyl)-1,2,3,4,5,6-hexahydro-1,5-methano-pyrido[1,2a][1,5]diazocin-8-one;
6-methyl-5-oxo-6,13-diazatetracyclo[9.3.1.0$^{2,10}$.0$^{4,8}$]pentadeca-2(10),3,8-triene;
5-oxo-6,13-diazatetracyclo[9.3.1.0$^{2,10}$.0$^{4,8}$]pentadeca-2(10),3,8-triene;
6-oxo-5,7,13-triazatetracyclo[9.3.1.0$^{2,10}$.0$^{4,8}$]pentadeca-2(10),3,8-triene;
4,5-difluoro-10-aza-tricyclo[6.3.1.0$^{2,7}$]dodeca-2(7),3,5-triene;
5-fluoro-10-aza-tricyclo[6.3.1.0$^{2,7}$]dodeca-2(7),3,5-triene-4-carbonitrile;
4-ethynyl-5-fluoro-10-aza-tricyclo[6.3.1.0$^{2,7}$]

dodeca-2(7),3,5-triene;

5-ethynyl-10-aza-tricyclo[6.3.1.0$^{2,7}$]dodeca-2(7),3,5-triene-4-carbonitrile;

6-methyl-5-thia-5-dioxa-6,13-diazatetracyclo[9.3.1.0$^{2,10}$.0$^{4,8}$]pentadeca-2(10),3,8-triene;

10-aza-tricyclo[6.3.1.0$^{2,7}$]dodeca-2(7),3,5-triene;

4-fluoro-10-aza-tricyclo[6.3.1.0$^{2,7}$]dodeca-2(7),3,5-triene;

4-methyl-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

7-methyl-5,7,13-triazatetracyclo[9.3.1.0$^{2,10}$.0$^{4,8}$]pentadeca-2(10),3,5,8-tetraene;

6-methyl-5,7,13-triazatetracyclo[9.3.1.0$^{2,10}$.0$^{4,8}$]pentadeca-2(10),3,5,8-tetraene;

6,7-dimethyl-5,7,13-triazatetracyclo[9.3.1.0$^{2,10}$.0$^{4,8}$]pentadeca-2(10),3,5,8-tetraene;

6-methyl-7-phenyl-5,7,13-triazatetracyclo[9.3.1.0$^{2,10}$.0$^{4,8}$]pentadeca-2(10),3,5,8-tetraene;

6,7-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

14-methyl-5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

4-chloro-10-azatricyclo[6.3.1.0$^{2,7}$]dodeca-2(7),3,5-triene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

10-azatricyclo[6.3.1.0$^{2,7}$]dodeca-2(7),3,5-trien-4-ol;

7-methyl-5-oxa-6,13-diazatetracyclo[9.3.1.0$^{2,10}$.0$^{4,8}$]pentadeca-2,4(8),6,9-tetraene;

4,5-dichloro-10-azatricyclo[6.3.1.0$^{2,7}$]dodeca-2(7),3,5-triene;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

5,7,14-triazatetracyclo[10.3.1.0$^{2,10}$.0$^{4,8}$]hexadeca-2(10),3,5,8-tetraene;

5,6-dimethyl-5,7,14-triazatetracyclo[10.3.1.0$^{2,10}$.0$^{4,8}$]hexadeca-2(10),3,6,8-tetraene;

5-methyl-5,7,14-triazatetracyclo[10.3.1.0$^{2,10}$.0$^{4,8}$]hexadeca-2(10),3,6,8-tetraene;

6-(trifluoromethyl)-7-thia-5,14-diazatetracyclo[10.3.1.0$^{2,10}$.0$^{4,8}$]hexadeca-2(10)3,58-tetraene;

5,8,15-triazatetracyclo[11.3.1.0$^{2,11}$.0$^{4,9}$]heptadeca-2(11),3,5,7,9-pentaene;

7-methyl-5,8,15-triazatetracyclo[11.3.1.0$^{2,11}$.0$^{4,9}$]heptadeca-2(11),3,5,7,9-pentaene;

6-methyl-5,8,15-triazatetracyclo[11.3.1.0$^{2,11}$.0$^{4,9}$]heptadeca-2(11),3,5,7,9-pentaene;

6,7-dimethyl-5,8,15-triazatetracyclo[11.3.1.0$^{2,11}$.0$^{4,9}$]heptadeca-2(11),3,5,7,9-pentaene;

7-oxa-5,14-diazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

5-methyl-7-oxa-6,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

7-methyl-5-oxa-6,14-diazatetracyclo[10.3.1.0$^{2,10}$.0$^{4,8}$]hexadeca-2(10),3,6,8-tetraene;

4,5-difluoro-11-azatricyclo[7.3.1.0$^{2,7}$]trideca-2(7),3,5-triene;

4-chloro-5-fluoro-11-azatricyclo[7.3.1.0$^{2,7}$]trideca-2(7),3,5-triene;

5-chloro-4-fluoro-11-azatricyclo[7.3.1.0$^{2,7}$]trideca-2(7),3,5-triene;

4-(1-ethynyl)-5-fluoro-11-azatricyclo[7.3.1.0$^{2,7}$]trideca-2(7),3,5-triene;

5-(1-ethynyl)-4-fluoro-11-azatricyclo[7.3.1.0$^{2,7}$]trideca-2(7),3,5-triene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

11-aza-tricyclo[7.3.1.0$^{2,7}$]trideca-2(7),3,5-trien-6-ol;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol;

4-nitro-11-aza-tricyclo[7.3.1.0$^{2,7}$]trideca-2(7),3,5-triene;

5-nitro-11-aza-tricyclo[7.3.1.0$^{2,7}$]trideca-2(7),3,5-triene;

5-fluoro-11-aza-tricyclo[7.3.1.0$^{2,7}$]trideca-2(7),3,5-triene;

6-hydroxy-5-methoxy-11-aza-tricyclo[7.3.1.0$^{2,7}$]trideca-2(7),3,5-triene

and a pharmaceutically acceptable salt and an optical isomer thereof.

8. The method according to claim 7, wherein the nicotine partial agonist is selected from

9-bromo-1,2,3,4,5,6-hexahydro-1,5-methano-pyrido[1,2-a][1,5]diazocin-8-one;

9-chloro-1,2,3,4,5,6-hexahydro-1,5-methano-pyrido[1,2-a][1,5]diazocin-8-one;

9-fluoro-1,2,3,4,5,6-hexahydro-1,5-methano-pyrido[1,2-a][1,5]diazocin-8-one;

9-acetyl-1,2,3,4,5,6-hexahydro-1,5-methano-pyrido[1,2a][1,5]diazocin-8-one;

9-iodo-1,2,3,4,5,6-hexahydro-1,5-methano-pyrido[1,2a][1,5]diazocin-8-one;

9-cyano-1,2,3,4,5,6-hexahydro-1,5-methano-pyrido[1,2a][1,5]diazocin-8-one;

9-carbomethoxy-1,2,3,4,5,6-hexahydro-1,5-methano-pyrido[1,2a][1,5]diazocin-8-one;

9-carboxyaldehyde-1,2,3,4,5,6-hexahydro-1,5-methano-pyrido[1,2a][1,5]diazocin-8-one;

9-(2,6-difluorophenyl)-1,2,3,4,5,6-hexahydro-1,5-methano-pyrido[1,2a][1,5]diazocin-8-one;

9-phenyl-1,2,3,4,5,6-hexahydro-1,5-methano-pyrido[1,2a][1,5]diazocin-8-one;

9-(2-fluorophenyl)-1,2,3,4,5,6-hexahydro-1,5-methano-pyrido[12a][1,5]diazocin-8-one;

6-methyl-5-thia-5-dioxa-6,13-diazatetracyclo[9.3.1.0$^{2,10}$.0$^{4,8}$]pentadeca-2(10),3,8-triene;

4-fluoro-10-aza-tricyclo[6.3.1.0$^{2,7}$]dodeca-2(7),3,5-triene;

4-trifluoromethyl-10-aza-tricyclo[6.3.1.0$^{2,7}$]dodeca-2(7),3,5-triene;

4-nitro-10-azatricyclo[6.3.1.0$^{2,7}$]dodeca-2(7),3,5-triene;

6-methyl-5,7,13-triazatetracyclo[9.3.1.0$^{2,10}$.0$^{4,8}$]pentadeca-2(10),3,5,8-tetraene;

67-dimethyl-5,8,14-triazatetracyclo[10.3.1.0$^{2,11}$.0$^{4,9}$]hexadeca-2(11),3,5,7,9-pentaene;

5,8,14-triazatetracyclo[10.3.1.0$^{2,11}$.0$^{4,9}$]hexadeca-2(11),3,5,7,9-pentaene;

5-oxa-7,13-diazatetracyclo[9.3.1.0$^{2,10}$.0$^{4,8}$]pentadeca-2(10),3,6,8-tetraene;

6-methyl-5-oxa-7,13-diazatetracyclo[9.3.1.0$^{2,10}$.0$^{4,8}$]pentadeca-2(10),3,6,8-tetraene;

10-azatricyclo[6.3.1.0$^{2,7}$]dodeca-2(7),3,5-trien-4-yl cyanide;

1-(10-azatricyclo[6.3.1.0$^{2,7}$]dodeca-2(7),3,5-trien-4-yl)-1-ethanone;

11-azatricyclo[7.3.1.0$^{2,7}$]trideca-2(7),3,5-triene-5-carbonitrile;

1-[11-azatricyclo[7.3.1.0$^{2,7}$]trideca-2(7),3,5-trien-5-yl]-1-ethanone;

1-[11-azatricyclo[7.3.1.0$^{2,7}$]trideca-2(7),3,5-trien-5-yl]-1-propanone;

4-fluoro-11-azatricyclo[7.3.1.0$^{2,7}$]trideca-2(7),3,5-triene-5-carbonitrile;

5-fluoro-11-azatricyclo[7.3.1.0$^{2,7}$]trideca-2(7),3,5-triene-4-carbonitrile;

6-methyl-7-thia-5,14-diazatetracyclo[10.3.1.0$^{2,10}$.0$^{4,8}$]hexadeca-2(10),3,5,8-tetraene;

6-methyl-5,7,14-triazatetracyclo[10.3.1.0$^{2,10}$.0$^{4,8}$]hexadeca-2(10),3,5,8-tetraene;

6,7-dimethyl-5,7,14-triazatetracyclo[10.3.1.0$^{2,10}$.0$^{4,8}$]hexadeca-2(10),3,5,8-tetraene;

6-methyl-7-oxa-5,14-diazatetracyclo[10.3.1.0$^{2,10}$.0$^{4,8}$]hexadeca-2(10),3,5,8-tetraene;

6-methyl-5-oxa-7,14-diazatetracyclo[10.3.1.0$^{2,10}$.0$^{4,8}$]hexadeca-2(10),3,6,8-tetraene;

5,6-difluoro-11-aza-tricyclo[7.3.1.0$^{2,7}$]trideca-2,4,6-triene;

6-trifluoromethyl-11-aza-tricyclo[7.3.1.0$^{2,7}$]trideca-2,4,6-triene;

6-methoxy-11-aza-tricyclo[7.3.1.0$^{2,7}$]trideca-2(7),3,5-triene;

6-fluoro-11-aza-tricyclo[7.3.1.0$^{2,7}$]trideca-2(7),3,5-triene;

11-aza-tricyclo[7.3.1.0$^{2,7}$]trideca-2(7),3,5-trien-5-ol; and the pharmaceutically acceptable salts and optical isomers thereof.

9. The method according to claim 5, wherein the nicotine receptor partial agonist and the anti-obesity agent or weight loss facilitator are administered substantially simultaneously.

10. A pharmaceutical composition for treating a disorder or condition selected from the group consisting of disorders and conditions in which obesity or an overweight condition predominates, including Type 2 diabetes mellitus, hypertension, dyslipidemia and increased mortality in a mammal, the method comprising:

(a) a nicotine receptor partial agonist or a pharmaceutically acceptable salt thereof;

(b) an anti-obesity agent or a weight loss promoter or facilitator or a pharmaceutically acceptable salt thereof; and

(c) a pharmaceutically acceptable carrier;

wherein the active agents "a" and "b" above are present in amounts that render the composition effective in treating such disorder or condition.

11. A method of treating a disorder or condition selected from the groups of disorders and conditions in which obesity or an overweight condition predominates in a mammal including Type 2 diabetes mellitus, hypertension, dyslipidemia and increased morality, the method comprising administering to said mammal:

(a) a nicotine receptor partial agonist ar a pharmaceutically acceptable salt thereof; and

(b) an anti-obesity agent or weight loss facilitation or a pharmaceutically acceptable salt thereof;

wherein the active agent "a" and "b" above are present in amounts that render the composition effective that render the composition effective in treating such disorder or condition.